# EUROPEAN PATENT APPLICATION

(11) **EP 4 478 090 A1**
(43) Date of publication of application: **18.12.2024**
(21) Application number: 22927386.7
(22) Date of filing: 16.02.2022
(51) Int. Cl.: G01T 1/20, G01T 1/161, A61B 6/00, C01G 29/00

(54) **X-RAY DETECTOR COMPRISING SCINTILLATOR, WHICH COMPRISES PEROVSKITE COMPOUND**

(71) Applicant: LG Electronics Inc., Yeongdeungpo-gu Seoul 07336 (KR)
(72) Inventor: LEE, Sungeun, Seoul 06772 (KR); PARK, Sangjun, Seoul 06772 (KR); ITO, Mikiko, Seoul 06772 (KR)
(74) Representative: Schornack, Oliver
(86) International application number: PCT/KR2022/002272
(87) International publication number: WO 2023/157986

(57) **Abstract**

An X-ray detector according to the present disclosure comprises: a scintillator for converting incident X-rays into visible rays; a photoelectric conversion part, which is disposed below the scintillator and converts the visible rays into electrical signals; and a substrate disposed below the photoelectric conversion part, wherein the scintillator comprises a perovskite compound represented by the following chemical formula 1. [Chemical Formula 1] A₃B₂X₅:Activator (In the chemical formula, A is a monovalent metal cation, B is a divalent metal cation, X is a monovalent anion, and the activator is thallium (Tl) or indium (In).)

## Description

### TECHNICAL FIELD

The present invention relates to an X-ray detector including a perovskite compound.

### BACKGROUND ART

Representative scintillator materials currently used in commercialized X-ray detectors are CsI(TI) and Gd₂O₂S:Tb(Gadox).

Since the CsI(TI) material has high X-ray generation and a wavelength of visible rays emitted by the doped Tl component is 550 nm, there is an advantage in that the coupled optical sensor has excellent quantum detection efficiency. However, since the CsI(TI) material exists in a crystalline state, crystals are broken when bent to significantly deteriorate efficiency, and there is a disadvantage in that since thermal evaporation equipment is used, manufacturing costs are expensive.

In addition, the Gd₂O₂S:Tb(Gadox) material has high X-ray luminous efficiency due to its high atomic number and density and is manufactured by mixing a binder and a dispersant, and thus, the manufacturing process is simple, and the price is low. However, the Gd₂O₂S:Tb(Gadox) material has spatial resolution that is significantly less than that of the Csl(TI) due to light scattering by powder particles.

Thus, a direct-type X-ray detector is required to maintain high resolution and spatial resolution without spreading or blurring an image due to the scattering of light by the scintillator. In addition, optimization of the thickness of the scintillator that meets the X-ray energy and spatial resolution that appropriate for the intended application fields is required.

In addition, generally, as a thickness of the scintillator increases, an amount of light generated by the X-ray absorption increases, but the spatial resolution is rapidly deteriorated due to the light scattering. Thus, research and development of the scintillator to increase in amount of light without a loss of the spatial resolution are required.

In addition, a composition of the existing scintillator is manufactured through a single crystal growth method. In this case, the composition of the single crystal scintillator has to be processed into a pixel size and arranged on a photodiode, but there is a problem in that a lot of time and money has be invested in the processing process.

Thus, there is a need for a technology that uniformly adjusts the scintillator on the photodiode while reducing the cost by shortening the process time.

### DISCLOSURE OF THE INVENTION

### TECHNICAL PROBLEM

An object of the present disclosure for solving the above problems is to provide an X-ray detector, in which a composition of a scintillator, which is realized through synthesis of chemical quantum dots (QD) is processed into a film form and then is attached on a photodiode or directly applied to be printed, thereby reducing a process time and cost.

### TECHNICAL SOLUTION

An X-ray detector according to an embodiment of the present disclosure includes: a scintillator configured to convert incident X-rays into visible rays; a photoelectric conversion part disposed below the scintillator to convert the visible rays into electrical signals; a substrate disposed below the photoelectric conversion part, wherein the scintillator comprises a perovskite compound represented by following chemical formula 1.

[Chemical Formula 1] A₃B₂X₅: Activator

(In the chemical formula, A is a monovalent metal cation, B is a divalent metal cation, X is a monovalent anion, and the activator is thallium (Tl) or indium (In).)

In addition, the X-ray detector according to an embodiment of the present disclosure may further include: a reflective layer disposed on the scintillator to reflect the visible rays downward; and a flexible substrate disposed on the reflective layer.

In addition, the monovalent metal cation of the X-ray detector according to an embodiment of the present disclosure may include at least one of Li⁺, Na⁺, K⁺, Rb⁺, Cs⁺, or Au(I)⁺.

In addition, the divalent metal cation of the X-ray detector according to an embodiment of the present disclosure may include at least one of Sn²⁺, Ge²⁺, Cu²⁺, Co²⁺, Ni²⁺, Ti²⁺, Zr²⁺, Hf²⁺, or Rf²⁺.

In addition, the monovalent anion of the X-ray detector according to an embodiment of the present disclosure may include at least one of F⁻, Cl⁻, Br⁻, I⁻, SCN⁻, or BF₄⁻.

In addition, the perovskite compound according to an embodiment of the present disclosure may include a nanocrystal particle.

In addition, the perovskite compound according to an embodiment of the present disclosure may be created through quantum dot (QD) synthesis.

In addition, a particle size of the nanocrystal particle according to an embodiment of the present disclosure may be in the range of 1 nm or more to 950 nm or less.

In addition, the scintillator according to an embodiment of the present disclosure may further include an organic binder.

In addition, the organic binder according to an embodiment of the present disclosure may include at least one of a polydimethyl siloxane resin, an acrylic resin, an ether resin, a polyvinyl acetate resin, a polystyrene resin, a polycarbonate resin, a polyamide resin, or a polyurethane resin.

In addition, in the scintillator according to an embodiment of the present disclosure, the perovskite compound and the organic binder may be contained in the scintillator at a weight ratio of 90:10 or 10:90.

In addition, a thickness of the scintillator according to an embodiment of the present disclosure may be in the range of 1 um or more to 1,000 um or less.

In addition, the photoelectric conversion part according to an embodiment of the present disclosure may include at least one of a silicon photodiode, a complementary metal oxide semiconductor, or an organic photodiode.

### ADVANTAGEOUS EFFECTS

In the X-ray detector according to the embodiment of the present disclosure, the composition of scintillator, which is realized through the synthesis of the chemical quantum dots (QD) is processed into the film form and then is attached on the photodiode or directly applied to be printed, thereby reducing the process time and cost.

### BRIEF DESCRIPTION OF THE DRAWINGS

FIG. 1 is a view an X-ray detector according to an embodiment of the present disclosure.
FIG. 2 is a view for explaining a scintillator according to an embodiment of the present disclosure.
FIG. 3 is a view for explaining a scintillator according to a related art.
FIG. 4 is a view for explaining an X-ray imaging system according to an embodiment of the present disclosure.

### MODE FOR CARRYING OUT THE INVENTION

Hereinafter, embodiments disclosed in this specification is described with reference to the accompanying drawings, and the same or corresponding components are given with the same drawing number regardless of reference number, and their duplicated description will be omitted. Furthermore, terms, such as a "module" ad a "unit", are used for convenience of description, and they do not have different meanings or functions in themselves. Moreover, detailed descriptions related to well-known functions or configurations will be ruled out in order not to unnecessarily obscure subject matters of the present disclosure. However, this does not limit the present disclosure within specific embodiments and it should be understood that the present disclosure covers all the modifications, equivalents, and replacements within the idea and technical scope of the present disclosure.

It will be understood that although the ordinal numbers such as first and second are used herein to describe various elements, these elements should not be limited by these numbers. The terms are only used to distinguish one component from other components.

It will also be understood that when an element is referred to as being ‴connected to" or "engaged with" another element, it can be directly connected to the other element, or intervening elements may also be present. It will also be understood that when an element is referred to as being 'directly connected to' another element, there is no intervening elements.

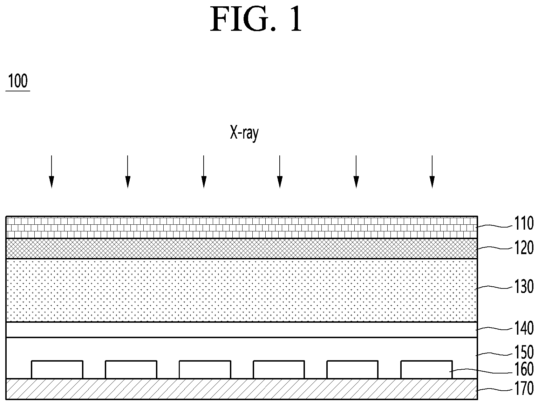

FIG. 1 is a view an X-ray detector according to an embodiment of the present disclosure.

Referring to FIG. 1, an X-ray detector 100 may include a flexible substrate 110, a reflective layer 120, a scintillator 130, an attachment layer 140, a protective layer 150, a photoelectric conversion part 160, and a substrate 170.

The flexible substrate 100 may be disposed on the reflective layer 120 to protect the X-ray detector 100 from an external impact or dust.

The reflective layer 120 may be disposed on top of the scintillator 130 to reflect visible rays converted by the scintillator 130 downward. For example, when X-rays are converted into visible rays in the scintillator 130, the converted visible rays may travel in various directions. In this case, when the converted visible rays travels upward in a direction opposite to the photoelectric conversion part 160, the reflective layer 120 may reflect the visible rays traveling upward to the downward direction in which the photoelectric conversion part 160 is disposed.

The scintillator 130 may convert the X-rays incident from the outside into the visible rays.

The attachment layer 140 may be disposed between the scintillator 130 and the photoelectric conversion part 160 to attach the scintillator 110 to the photoelectric conversion part 160.

The protective layer 150 may be disposed on the photoelectric conversion part 160 to protect the photoelectric conversion part 160. The photoelectric conversion part 160 may be embedded in the protective layer 150 and protected by the protective layer 150. The protective layer 125 may be made of an insulating material.

The photoelectric conversion part 160 may include at least one of a silicon photodiode, a complementary metal-oxide semiconductor (CMOS), or an organic photodiode.

The silicon photodiode, the complementary metal-oxide semiconductor (CMOS), and the organic photodiode of the photoelectric conversion part 160 may convert the visible rays into electrical signals.

The substrate 170 may be disposed below the photoelectric conversion part 160. The photoelectric conversion part 160 may be disposed on the substrate 170. The substrate 170 may include a thin film transistor array (TFT array).

The scintillator 130 may convert the X-rays incident from the outside into the visible rays.

The scintillator 130 may include a perovskite compound.

Perovskite may include a perovskite compound represented by the following chemical formula 1.

[Chemical Formula 1] A₃B₂X₅: Activator

In the above chemical formula, A may be a monovalent metal cation, B may be a divalent metal cation, and X may be a monovalent anion. In addition, the activator may include one of thallium (Tl) and indium (In).

The monovalent metal cation A may include at least one of Li⁺, Na⁺, K⁺, Rb⁺, Cs⁺, or Au(I)⁺.

The divalent metal cation B may include at least one of Sn²⁺, Ge²⁺, Cu²⁺, Co²⁺, Ni²⁺, Ti²⁺, Zr²⁺, Hf²⁺, or Rf²⁺.

the monovalent anion X may include at least one of F⁻, Cl⁻, Br⁻, I⁻, SCN⁻, or BF₄⁻.

The perovskite compound may include a nanocrystal particle. A particle size of the nanocrystal particle may be in the range of 1 nm or more to 950 nm or less.

The nanocrystal particle may be a nano-sized crystalline particle and may be quantum dot (QD) particle. The perovskite compound may be a compound created through quantum dot (QD) synthesis.

For example, the perovskite compound forming the scintillator 130 may be thallium doped cesium copper Iodide (Cs₃Cu₂I₅:Tl) and may include quantum dot particles. In the case of the thallium doped cesium copper Iodide (Cs₃Cu₂I₅:Tl), a composition compound for the scintillator obtained through the quantum dot synthesis, the composition compound may be implemented in the form of a film and attached to the attachment layer 140. In addition, the thallium doped cesium copper Iodide (Cs₃Cu₂I₅:Tl), which is the composition compound for the scintillator obtained through the quantum dot synthesis, may be directly applied on the attachment layer 140, the protective layer 150, or the photoelectric conversion part 160 through printing.

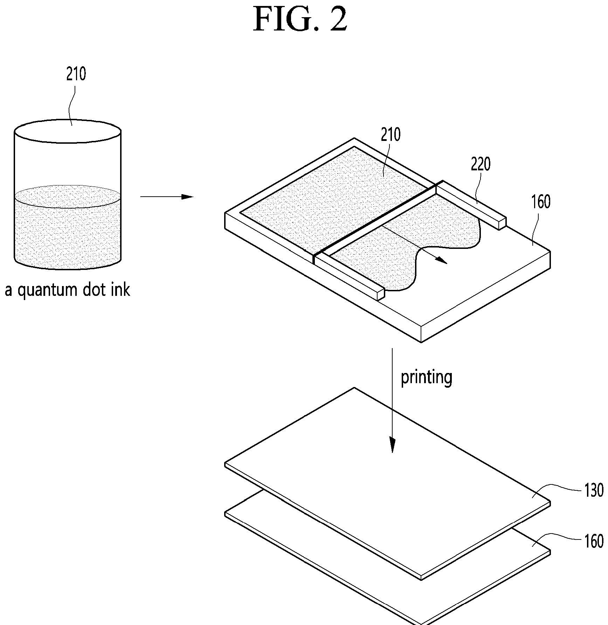

FIG. 2 is a view for explaining the scintillator according to an embodiment of the present disclosure.

Referring to FIG. 2, the perovskite compound included in the scintillator 130 may be quantum dot ink 210 created by the quantum dot particles, which are nano-sized crystalline particles.

The quantum dot ink 210 may be directly applied through the printing on the attachment layer 140, the protective layer 150, or the photoelectric conversion part 160 using a printing device 220.

In this case, the scintillator 130 may be uniformly applied on the photoelectric conversion part 160 over a large area. Thus, it has the advantage of shortening a process time, improving productivity, and securing price competitiveness compared to a vacuum deposition method, which is one of the existing scintillator manufacturing methods.

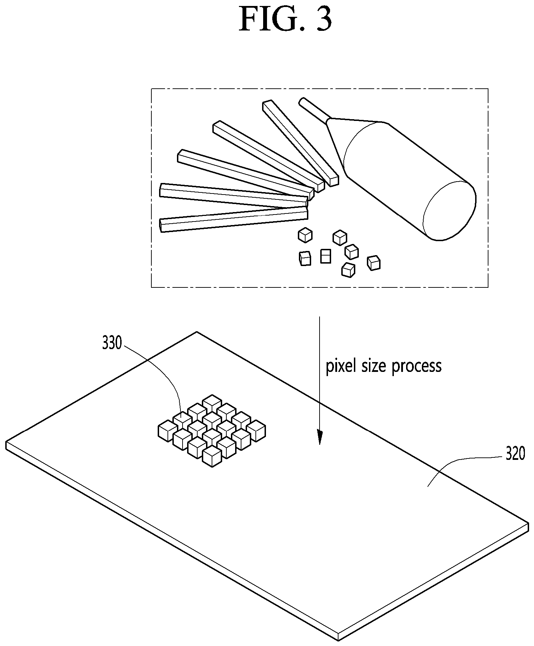

FIG. 3 is a view for explaining a scintillator according to a related art.

Referring to FIG. 3, a compound 310 included in the scintillator according to the related art may be a compound manufactured through a single crystal growth method. The single crystal scintillator composition 310 may be processed into a pixel size of the photoelectric conversion part 320 so that the scintillator 330 is implemented in such a manner in which the composition 310 is in contact with the photoelectric conversion part.

In this case, it is difficult to arrange the single crystal scintillator composition 310 uniformly over the large area, and there is a problem that a lot of time and money has to be invested in the processing process of the scintillator 330.

The scintillator 110 may further include an organic binder. The organic binder may be included in the scintillator 130 together with the perovskite compound to improve the flexibility of the scintillator 130.

The organic binder may include at least one of a polydimethyl siloxane resin, an acrylic resin, an ether resin, a polyvinyl acetate resin, a polystyrene resin, a polycarbonate resin, a polyamide resin, or a polyurethane resin.

The scintillator 130 may include the perovskite compound and the organic binder at a weight ratio of 90:10 or 10:90. Thus, the resolution of the scintillator 130 may be improved.

A thickness of the scintillator 130 may be in a range from 1 um to 1,000 um. If the thickness of the scintillator 130 exceeds the above range, the visible rays scattered from the scintillator 130 may increase, and the resolution decrease, and if the thickness of the scintillator 130 is less than the above range, there is a problem that an amount of X-rays to be absorbed may be reduced.

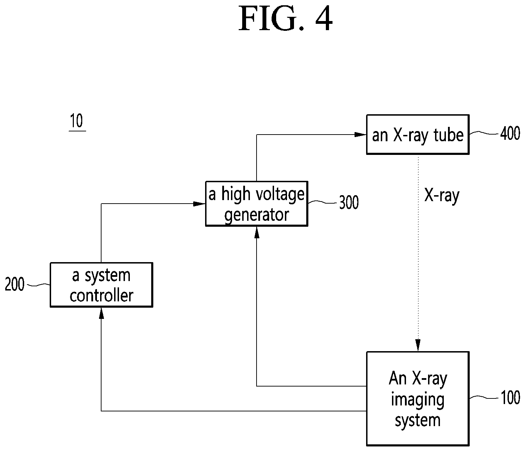

FIG. 4 is a diagram for explaining an X-ray imaging system according to an embodiment of the present disclosure.

An X-ray imaging system 10 may include an X-ray detector 100, a system controller 200, a high voltage generator 300, and an X-ray tube 400.

The X-ray detector 100 may generate an electrical signal corresponding to a dose of the transmitted X-rays to generate X-ray image data. The X-ray detector 100 may transmit the X-ray image data to the system controller 200.

The system controller 200 may set X-ray irradiation conditions. The system controller 200 may set X-ray irradiation conditions including a tube voltage, tube current, and an X-ray irradiation time. The system controller 200 may transmit the set X-ray irradiation conditions to the high voltage generator 300.

The high voltage generator 300 may apply the tube voltage and the tube current to the X-ray tube 400 for a set irradiation time based on the set X-ray irradiation conditions.

The X-ray tube 400 may receive the tube voltage and the tube current from the high voltage generator 300 to irradiate the X-rays toward the X-ray detector 100.

The X-ray detector 100 may generate an X-ray sensing signal corresponding to current generated when the X-ray is incident and amplify the generated X-ray sensing signal to transmit the X-ray sensing signal to the high voltage generator 300. The X-ray sensing signal may be a signal for monitoring the X-ray irradiation.

The high voltage generator 300 may determine whether the currently incident X-ray dose exceeds a critical dose based on the amplified X-ray sensing signal. If the X-ray dose exceeds the critical dose, the tube voltage and tube current applied to the X-ray tube 400 may be stopped.

The system controller 200, the high voltage generator 300, and the X-ray tube 400 may transmit and receive data through wired communication. In addition, the system controller 200, the high pressure generator 300, and the X-ray detector 100 may transmit and receive data through wired or wireless communication.

The above-disclosed subject matter is to be considered illustrative, and not restrictive, and the appended claims are intended to cover all such modifications, enhancements, and other embodiments, which fall within the scope of the present disclosure.

Thus, the embodiment of the present disclosure is to be considered illustrative, and not restrictive, and the present disclosure is not limited to the foregoing embodiment.

Therefore, the scope of the present disclosure is defined not by the detailed description of the invention but by the appended claims, and all differences within the scope will be construed as being included in the present disclosure.

## Claims

1. An X-ray detector comprising:
a scintillator configured to convert incident X-rays into visible rays;
a photoelectric conversion part disposed below the scintillator to convert the visible rays into electrical signals;
a substrate disposed below the photoelectric conversion part,
wherein the scintillator comprises a perovskite compound represented by following chemical formula 1.
[Chemical Formula 1] A₃B₂X₅:Activator
(In the chemical formula 1, A is a monovalent metal cation, B is a divalent metal cation, X is a monovalent anion, and the activator is thallium (Tl) or indium (In))

2. The X-ray detector according to claim 1, further comprising:
a reflective layer disposed on the scintillator to reflect the visible rays downward; and
a flexible substrate disposed on the reflective layer.

3. The X-ray detector according to claim 1, wherein the A comprises at least one of Li⁺, Na⁺, K⁺, Rb⁺, Cs⁺, or Au(I)⁺.

4. The X-ray detector according to claim 1, wherein the B comprises at least one of Sn²⁺, Ge²⁺, Cu²⁺, Co²⁺, Ni²⁺, Ti²⁺, Zr²⁺, Hf²⁺, or Rf²⁺.

5. The X-ray detector according to claim 1, wherein the X comprises at least one of F⁻, Cl⁻, Br⁻, I⁻, SCN⁻, or BF₄⁻.

6. The X-ray detector according to claim 1, wherein the perovskite compound is a nanocrystal particle.

7. The X-ray detector according to claim 6, wherein the perovskite compound is created through quantum dot (QD) synthesis.

8. The X-ray detector according to claim 6, wherein a particle size of the nanocrystal particle is in the range of 1 nm or more to 950 nm or less.

9. The X-ray detector according to claim 1, wherein the scintillator further comprises an organic binder.

10. The X-ray detector according to claim 9, wherein the organic binder comprises at least one of a polydimethyl siloxane resin, an acrylic resin, an ether resin, a polyvinyl acetate resin, a polystyrene resin, a polycarbonate resin, a polyamide resin, or a polyurethane resin.

11. The X-ray detector according to claim 9, wherein the perovskite compound and the organic binder are contained in the scintillator at a weight ratio of 90:10 or 10:90.

12. The X-ray detector according to claim 1, wherein a thickness of the scintillator is in the range of 1 um or more to 1,000 um or less.

13. The X-ray detector according to claim 1, wherein the photoelectric conversion part comprises at least one of a silicon photodiode, a complementary metal oxide semiconductor, or an organic photodiode.
